# EUROPEAN PATENT SPECIFICATION

(11) **EP 1 662 008 B1**
(45) Date of publication and mention of the grant of the patent: **16.12.2009**
(21) Application number: 05015561.3
(22) Date of filing: 18.07.2005
(51) Int. Cl.: C12Q 1/68, B01L 3/00

(54) **Apparatus for and method of purifying nucleic acids by using beads with different laser absorption**
Gerät und Verfahren zur Aufreinignug von Nukleinsäuren mittels Partikel mit unterschiedlichen Laserabsorptionseigenschaften
Appareil et procédé pour la purification d'acide nucléique utilisant des microspherès ayant les caractéristiques d'absorption à laser differentes

(30) Priority: 25.11.2004 KR 2004097601
(43) Date of publication of application: 31.05.2006
(73) Proprietor: Samsung Electronics Co., Ltd, Suwon-si, Gyeonggi-do (KR)
(72) Inventor: Lee, Jeong-gun, Seoul (KR); Kwon, Young-nam 104-306, Gunpo-si Gyeonggi-do (KR); Lee, Myo-yong 306-1102 LG Xi Apt., Suwon-si Gyeonggi-do (KR); Yoo, Shin-i 101-2102 Ssangyong Apt., Gunpo-si Gyeonggi-do (KR); Cho, Yeon-ja 1-132 Jeonnong 3-dong, Seoul (KR); Kim, Young-a 245-1804, Yeongtong-gu Suwon-si Gyeonnggi-do (KR)
(74) Representative: Grünecker, Kinkeldey, Stockmair & Schwanhäusser Anwaltssozietät

(56) References cited:
- EP-A- 1 650 297
- EP-A- 1 655 366
- US-A1- 2003 096 429
- TAYLOR M T ET AL: "LYSING BACTERIAL SPORES BY SONICATION THROUGH A FLEXIBLE INTERFACE IN A MICROFLUIDIC SYSTEM" ANALYTICAL CHEMISTRY, AMERICAN CHEMICAL SOCIETY. COLUMBUS, US, vol. 73, no. 3, 1 February 2001 (2001-02-01), pages 492-496, XP001060095 ISSN: 0003-2700
- ELGORT MARC G ET AL: "Extraction and amplification of genomic DNA from human blood on nanoporous aluminum oxide membranes." CLINICAL CHEMISTRY OCT 2004, vol. 50, no. 10, October 2004 (2004-10), pages 1817-1819, XP002425504 ISSN: 0009-9147
- HOFMANN O ET AL: "LASER BASED DISRUPTION OF BACILLUS SPORES ON A MICROCHIP" SPECIAL PUBLICATION - ROYAL SOCIETY OF CHEMISTRY, ROYAL SOCIETY OF CHEMISTRY, LONDON, GB, 2005, pages 258-260, XP009066976 ISSN: 0260-6291

<110> Samsung Electronics Co. Ltd.
<120> Apparatus for and method of purifying nucleic acids by different laser absorption of beads
<130> EP35370FZpau
<140> 05 015 561.3
   <141> 2005-07-18
<150> 10-2004-0097601
   <151> 2004-11-25
<160> 2
<170> KopatentIn 1.71
<210> 1
   <211> 19
   <212> DNA
   <213> Artificial Sequence
<220>
   <223> forward primer
<400> 1
   agtgtggatt cgcactcct 19
<210> 2
   <211> 23
   <212> DNA
   <213> Artificial sequence
<220>
   <223> reverse primer
<400> 2
   gagttcttct tctaggggac ctg 23

## Description

### BACKGROUND OF THE INVENTION

### 1. Field of the Invention

The present invention relates to an apparatus for and method of purifying nucleic acids by different laser absorption of beads.

### 2. Description of the Related Art

An efficient extraction of DNA from cells is necessary for many applications and is essential for molecular diagnostics, specifically for pathogen identification and quantification. Molecular diagnostics is generally performed by DNA amplification after DNA extraction steps. DNA amplification reactions include polymerase chain reaction (PCR), ligase chain reaction, stranded-displacement amplification, nucleic acid-based amplification, repair chain reaction, helicase chain reaction, QB replicase amplification, ligation activated transcription.

Generally, isolation methods of DNA from cells use materials that have the proclivity of binding DNA. Some examples of these materials are silica, glass fiber, anion exchange resins and magnetic beads (Rudi, K. et al., Biotechniqures 22, 506-511 (1997); and Deggerdal, A. et al., Biotechniqures 22, 554-557 (1997)). To avoid the manual steps and to remove an operator error, several automatic machines have been developed for high-throughput DNA extraction.

Cell lysis is conventionally performed by mechanical, chemical, thermal, electrical, ultrasonic and microwave methods (Michael T. Taylor et al., Anal.Chem., 73, 492-496 (2001)).

Laser has many advantages for disruption of cells and is highly applicable to a Lab-On-a-Chip (LOC) (Huaina Li et al., Anal Chem, 73, 4625-4631 (2001)).

U.S. Patent Publication No. 2003/96429 A1 discloses a laser-induced cell lysis system. When only a laser beam is used, an efficient cell lysis does not occur. As a result of performing an experiment using E. coli placed in a very clear solution, it has been confirmed that when irradiating only a laser beam, a low cell lysis efficiency is obtained. A concentration of DNA measured after irradiating a laser for 150 seconds is 3.77 ng/µℓ because the laser energy is not efficiently transferred to the cells. A concentration of DNA measured after boiling cells at 95°C for 5 minutes by means of a conventional heating method is 6.15 ng/µℓ.

U.S. Patent No. 6,685,730 discloses optically-absorbing nanoparticles for enhanced tissue repair. This patent includes a method of joining tissue comprising: delivering nanoparticles having dimensions of from 1 to 1000 nanometers that absorb light at one or more wavelengths to the tissue to be joined; and exposing said nanoparticles to light at one or more wavelengths that are absorbed by the nanoparticles. This method causes only a loss of function of cells by using a laser beam and nanoparticles and there is no description of a method of disrupting cells by vibrating a solution containing cells and particles.

Conventionally, a method of purifying nucleic acids using a solid phase is known. For example, U.S. Patent No. 5,234,809 discloses a method of purifying nucleic acids using a nucleic acid binding solid phase. Specifically, the method includes mixing a starting material, a chaotropic material and a nucleic acid binding solid phase, separating the solid phase with the nucleic acid bound thereto from the liquid, and washing the solid phase nucleic acid complexes.

EP-A-1655366 and EP-A-1650297 both disclose apparatuses for purifying nucleic acids from cell lysates in which magnetic particles are added to a capillary together with the cell sample. Both documents are published after the present application's filing date.

However, this method is time consuming and complicated, and thus is not suitable for a LOC. The method also has a problem regarding the use of the chaotropic material. That is, when the chaotropic material is not used, nucleic acids are not bound to the solid phase. The chaotropic material is harmful to humans, and thus should be handled with caution. Also, the chaotropic material acts as a material inhibiting the subsequent processes, such as PCR, and thus should be removed from purified nucleic acids during or after purification.

For the purpose of LOC implementation, the purification process of nucleic acids after cell lysis is required for efficient PCR amplification. However, a conventional purification process of nucleic acids is time-consuming and has a problem of the use of separate chemicals. Thus, a method of rapidly and efficiently purifying nucleic acids without using separate chemicals is required.

Thus, the inventors of the present invention tried to develop a method to overcome the above problems and discovered that nucleic acids can be efficiently purified by using different laser absorption of beads when a magnetic bead with high laser absorption is used for cell lysis and a silicon bead or a silicon substrate with low laser absorption is used for nucleic acid purification.

### SUMMARY OF THE INVENTION

The present invention provides an apparatus for and method of purifying nucleic acids by different laser absorption of beads.

According to an aspect of the present invention, there is provided a nucleic acid purification apparatus for cells or viruses, as defined in claim 1 including: a cell lysis capillary having a sample inlet through which samples, magnetic beads, and a solid support are introduced; a vibrator attached to the capillary and mixing the samples, magnetic beads, and solid support in the capillary; a laser generator attached to the capillary and irradiating a laser beam onto the capillary; a magnetic force generator attached to the capillary and fixing the magnetic beads to a capillary wall; a waste chamber attached to the capillary and discharging a lysate; an elution buffer chamber attached to the capillary and eluting nucleic acids from the solid support having nucleic acids bound thereto; and a neutralization buffer chamber attached to the capillary and supplying a neutralization buffer for neutralizing an eluted nucleic acid solution.

According to another aspect of the present invention, there is provided a method of purifying nucleic acids as defined in claim 7 using the nucleic acid purification apparatus, the method including: injecting a solution containing cells or viruses in a capillary-shaped container containing magnetic beads and a solid support; operating a vibrator to mix the solution, the magnetic beads and the solid support; irradiating a laser beam onto the magnetic beads to disrupt the cells or viruses and binding compounds in the resulting cell or virus lysate to the magnetic beads and binding nucleic acids in the lysate to the solid support; fixing the magnetic beads, to which the compounds in the cell or virus lysate are bound, to a capillary-shaped container wall by means of a magnetic force generator; discharging the lysate which contains no magnetic bead; and eluting nucleic acids from the solid support and neutralizing them.

According to another aspect of the present invention, there is provided a method of continuously performing purification and amplification as defined in claim 8 of the nucleic acids using the nucleic acid purification apparatus, the method including: injecting a solution containing cells or viruses to a capillary-shaped container containing magnetic beads and a solid support; operating a vibrator to mix the solution, the magnetic beads, and the solid support; irradiating a laser beam onto the magnetic beads to disrupt the cells or viruses and binding compounds in the resulting cell or virus lysate to the magnetic beads and binding nucleic acids in the lysate to the solid support; fixing the magnetic beads, to which the compounds in the cell or virus lysate are bound, to a capillary-shaped container wall by means of a magnetic force generator; discharging the lysate which contains no magnetic bead; eluting the nucleic acids from the solid support and neutralizing them; and obtaining a solution that contains nucleic acids and transferring the resulting solution to a amplification chamber through a channel connecting the container and the amplification chamber to perform amplification.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic diagram of a system where a magnetic bead phase having PCR inhibitors bound thereto and a solid support phase having nucleic acids bound thereto are separated after lysing cells using magnetic beads and a laser beam;
FIG. 2A is a schematic diagram of a betaine-coated silica bead, which captures nucleic acids, and FIG. 2B is a schematic diagram of a betaine-coated silicon substrate in a pillar form, which captures nucleic acids;
FIG. 3 shows the results of electrophoresis of PCR products according to DNA purification methods;
FIG. 4 shows the concentrations of amplified PCR products according to DNA purification methods; and
FIG. 5 shows the concentrations of dimers produced by a PCR.

### DETAILED DESCRIPTION OF THE INVENTION

Hereinafter, the present invention will be described in more detail.

The present invention relates to a nucleic acid purification apparatus of cells or viruses, including: a cell lysis capillary having a sample inlet through which samples, magnetic beads, and a solid support are introduced; a vibrator attached to the capillary and mixing the samples, magnetic beads, and solid support in the capillary; a laser generator attached to the capillary and irradiating a laser beam onto the capillary; a magnetic force generator attached to the capillary and fixing the magnetic beads to a capillary wall; a waste chamber attached to the capillary and discharging a lysate; an elution buffer chamber attached to the capillary and eluting nucleic acids from the solid support having nucleic acids bound thereto; and a neutralization buffer chamber attached to the capillary and supplying a neutralization buffer for neutralizing the eluted nucleic acid solution.

A variety of beads have different laser absorption abilities. A magnetic bead absorbs a laser beam, but a solid support, such as a silica bead or a silicon substrate, passes a laser beam at a near-infrared wavelength without absorption. Thus, a laser absorption difference between two beads is produced, which causes a difference in heat absorption. Therefore, the magnetic bead can be used for cell lysis and the solid support, such as a silica bead or silicon substrate, can be used for nucleic acid purification.

In the apparatus, samples, magnetic beads, and a solid support for capturing nucleic acids injected through a sample inlet are mixed in the cell lysis capillary and cells are lysed when a laser beam is irradiated thereto. The cell lysis capillary may be composed of a material through which a laser beam can pass or have a window of such a material. The capillary may have a ratio of diameter to length ranging from 1:2 to 1:50 and have a diameter ranging from 1 nm to 5 mm. The capillary should be composed of a material to which magnetic beads can be effectively fixed. Examples of such a material include polymers, organic materials, silicon, glass and metals.

The vibrator is a device for mixing samples, magnetic beads, and the solid support in the cell lysis capillary and can be any device capable of vibrating.

The laser generator is a device for irradiating a laser beam onto the cell lysis capillary and can emit light with specific wavelengths. If the laser power is too low, the laser ablation cannot efficiently occur. The laser power is from 10 mW to 300 W for the continuous wave (CW) laser and 1 mJ/pulse to 1J/pulse for the pulse laser. The pulse laser may be 32 mJ/pulse to 1J/pulse and the CW laser has the power from 10 W to 300 W. When the CW is less than 10 mW and the pulse laser is less than 1 mJ/pulse, an energy sufficient to disrupt cells is not transferred. When the CW is greater than 300 W and the pulse laser is greater than 1 J/pulse, DNA is damaged.

Even though the magnetic beads having PCR inhibitors bound thereto are spontaneously attached to the capillary wall, a part of the magnetic beads cannot be attached to the capillary wall. Thus, the magnetic force generator is a device for supplying a magnetic force in order to fix such magnetic beads to the capillary wall.

The waste chamber discharges a cell lysate present in the cell lysis capillary after PCR inhibitors are bound to magnetic beads and nucleic acids are bound to the solid support. Since many PCR inhibitors may remain in the cell lysate, this discharge through the waste chamber is carried out in order to remove them.

After the cell lysate is discharged through the waste chamber, the solid support having nucleic acids bound thereto remains at a lower portion of the capillary. The elution buffer chamber is a chamber for supplying an elution buffer to separate nucleic acids from the solid support. The elution buffer includes a NaOH solution.

Since the eluted nucleic acids are denatured and unstable due to the high pH of the elution buffer, the neutralization buffer chamber is for supplying a neutralization buffer to stabilize them. The neutralization buffer includes a Tris buffer.

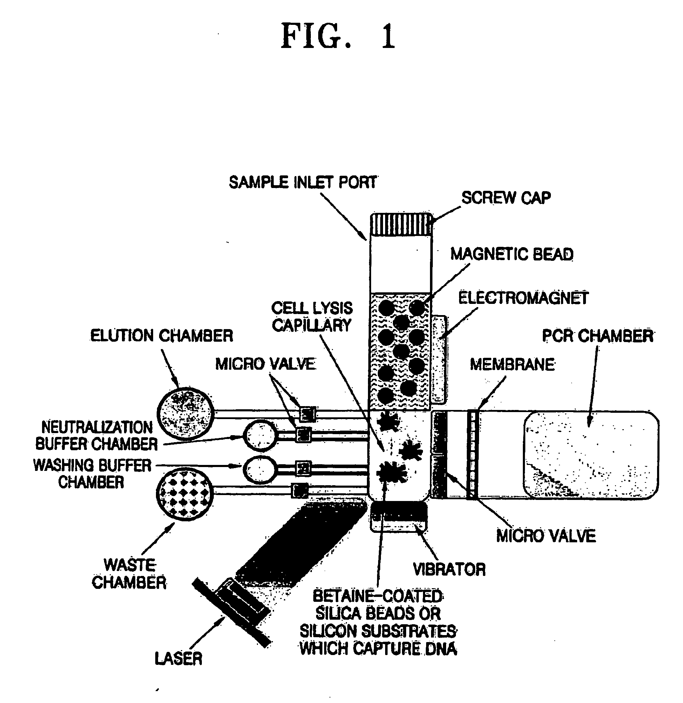

FIG. 1 is a schematic diagram of a system where a magnetic bead phase having PCR inhibitors bound thereto and a solid support phase having nucleic acids bound thereto are separated after lysing cells using magnetic bead and a laser. As shown in FIG. 1, after cell lysis, magnetic beads having PCR inhibitors bound thereto are boiled up due to laser absorption and attach to an upper portion of the capillary. The solid support, such as a silica bead or silicon substrate, through which a laser beam passes, captures nucleic acids, which are separated from the cell lysate, and is placed at a lower portion of the capillary. As a result, a phase separation occurs.

In an embodiment of the present invention, the vibrator may include sonicators, vibrators using a magnetic field, vibrators using an electric field, mechanical vibrators such as a vortex etc., or piezoelectric materials. The vibrator is attached to the cell lysis capillary and can be any device capable of vibrating the mixture of the cells or viruses, magnetic beads, and the solid support.

In an embodiment of the present invention, the magnetic force generator is located above a laser beam pathway and may be an electromagnet that is turned on when the magnetic beads in the cell lysis capillary are boiled. As illustrated in FIG. 1, the electromagnet should be located above the laser beam pathway because if it is located within the laser beam pathway, the magnetic beads are attached to the electromagnet prior to the magnetic beads absorbing the PCR inhibitors, thereby resulting in a reduction in the effects of adsorbing the PCR inhibitors. The electromagnet may be turned on when the magnetic beads in the cell lysis capillary are boiled. Although an electromagnetic is turned on before the magnetic beads are boiled, the magnetic force does not influence the magnetic beads due to the spatial separation of the magnetic bead and the electromagnet so that the magnetic beads cannot be attached to the electromagnet. In addition, beads should be magnetized in order to be removed by the electromagnet.

In an embodiment of the present invention, the nucleic acid purification apparatus may further include a DNA amplification chamber connected to the cell lysis capillary through a channel which is opened or closed by a valve. For the purpose of the LOC implementation, an amplification system of the purified DNA is necessary. The purified DNA can be detected using a spectrophotometer, micro magnetic beads, an electrochemical method, electrochemiluminescence, radiation and fluorescent label, a real-time PCR method, and the like. The PCR method is most suitable to sufficiently amplify the desired DNA. Other DNA amplification methods can also be applied and direct detection through the real-time PCR method, etc. is also possible.

In an embodiment of the present invention, the nucleic acid purification apparatus may further include a membrane which is located in a channel disposed between the cell lysis capillary and the DNA amplification chamber and filters the solid support. After the solid support used to capture nucleic acids discharges nucleic acids by means of the elution buffer, only nucleic acids should be transferred to the DNA amplification chamber and the solid support should be removed. Thus, in order to exclude the eluted solid support and transfer only the eluted nucleic acids to the DNA amplification chamber, a membrane for filtering the solid support is required. The membrane is not particularly restricted as long as it allows nucleic acids to pass and can filter the solid support.

In an embodiment of the present invention, the nucleic acid purification apparatus may further include a washing buffer chamber attached to the capillary and washing the solid support having nucleic acids bound thereto. After cells or viruses are lysed, a part of PCR inhibitors are bound to magnetic beads, and then the magnetic beads attach to the magnetic force generator to be removed, and nucleic acids attach to the solid support. Then, a process of removing impurities, which may have remained in the solid support, by washing the remaining solid support, to which nucleic acids are bound, after discharging the cell lysate through the waste chamber, is required. When this process is performed, nucleic acids are further purified to improve DNA amplification efficiency. The washing buffer includes a phosphate buffered saline (PBS), which can remove impurities without eluting nucleic acids from the solid support, but is not limited thereto.

The present invention also relates to a method of purifying nucleic acids using the nucleic acid purification apparatus, the method including: injecting a solution containing cells or viruses in a capillary- shaped container containing magnetic beads and a solid support; operating a vibrator to mix the solution, the magnetic beads and the solid support; irradiating a laser beam onto the magnetic beads to disrupt the cells or viruses and binding compounds in the resulting cell or virus lysate to the magnetic beads and binding nucleic acids in the lysate to the solid support; fixing the magnetic beads, to which the compounds in the cell or virus lysate are bound, to a capillary-shaped container wall by means of a magnetic force generator; discharging the lysate containing which contains no magnetic bead; and eluting nucleic acids from the solid support and neutralizing them.

In the method of the present invention, a difference in a laser absorbing ability of different types of beads is used. A magnetic bead absorbs a laser beam, but a solid support, such as a silica bead or a silicon substrate, passes a laser beam at near-infrared wavelength without absorption. Thus, a difference in laser absorption between two beads is produced. Therefore, the magnetic bead is used for cell lysis and the solid support, such as a silica bead or silicon substrate, is used for nucleic acid purification.

In this method, samples, magnetic beads and the solid support are injected to cell lysis capillary and a laser beam is irradiated onto them while mixing them using a vibrator. The magnetic beads absorb the laser beam to disrupt cells or viruses. The magnetic beads boiled up by the laser beam capture PCR inhibitors and are spontaneously attached to a capillary wall or attached to a capillary wall by means of a magnetic force generator. Nucleic acids in the cell lysate are bound to the solid support, such as a silica bead or silicon substrate and located at a lower portion of the capillary. Then, the solution was discharged through a waste chamber while remaining only the solid support with nucleic acids bound thereto in the cell lysis capillary. The solid support with nucleic acids bound thereto is then washed with a washing buffer to further remove PCR inhibitors. The nucleic acids bound to the solid support are eluted using an elution buffer and denatured nucleic acids are neutralized using a neutralization buffer to further purify nucleic acids. The obtained nucleic acid solution is transferred to a PCR chamber to amplify nucleic acids.

If a laser beam is irradiated onto a solution containing magnetic beads, a laser ablation occurs so that a shock wave, vapor pressure and heat are transferred to the cell surface. At this time, physical shocks are also applied to the cell surface. The laser ablation refers to general phenomenon occurred in materials exposed to a laser beam. The laser ablation rapidly raises the temperature of a material surface from several hundred to several thousand degrees. If the temperature of the material surface is raised to the evaporation point or higher, the saturated vapor pressure on the surface rapidly increases according to an evaporation of the liquid phase material.

The magnetic beads heated by the laser raise the temperature of the solution and directly disrupt cells. The magnetic beads in the solution do not act as a simple heat conductor but thermal, mechanical and physically influence the cell surface, thereby effectively disrupting the cell surface. The lysate of disrupted cells or viruses includes compounds which inhibit a PCR. Thus, to efficiently perform the PCR, a separate step for removing PCR inhibitors from the lysate is required, which is not suitable to efficiently implement LOC. In the method of the present invention, the magnetic beads with the PCR inhibitors attached thereto are fixed to a cell lysis capillary wall by means of the magnetic force generator and nucleic acids are more effectively purified by binding them to the solid support, thereby facilitating PCR.

Specifically, the magnetic beads to which PCR inhibitors, such as proteins denatured and cell debris, are attached are boiled by the energy of a laser to attach to a upper portion of the container wall and a phase of solid support having nucleic acids bound thereto without magnetic beads is located at a lower portion of the container, thereby easily removing the PCR inhibitors. This phase separation occurs more efficiently in a capillary with a limited diameter. A fixed electromagnet or permanent magnet can be used to effectively fix the separated phases and designate fixing regions.

The present invention also relates to a method of continuously performing purification and amplification of the nucleic acids using the nucleic acid purification apparatus, the method including: injecting a solution containing cells or viruses to a capillary-shaped container containing magnetic beads and a solid support; operating a vibrator to mix the solution, the magnetic beads, and the solid support; irradiating a laser beam onto the magnetic beads to disrupt the cells or viruses and binding compounds in the resulting cell or virus lysate to the magnetic beads and binding nucleic acids in the lysate to the solid support; fixing the magnetic beads, to which the compounds in the cell or virus lysate are bound, to a capillary-shaped container wall by means of a magnetic force generator; discharging the lysate which contains no magnetic bead; eluting the nucleic acids from the solid support and neutralizing them; and obtaining a solution that contains nucleic acids and transferring the resulting solution to a amplification chamber through a channel connecting the container and the amplification chamber to perform amplification.

For the purposes of LOC implementation, it is necessary to continuously perform isolation, purification, and amplification of nucleic acids. Thus, this purpose can be achieved by directly transferring the solution of DNA purified to the amplification chamber through the channel connecting the capillary-shaped container and the amplification container and then amplifying nucleic acids. The transferring of nucleic acids to the amplification chamber can be carried out by a pump using a mechanical force, etc.

In an embodiment of the present invention, the laser can be a pulse laser or a continuous wave (CW) laser.

If the laser power is too low, the laser ablation cannot efficiently occur. The laser power is from 10 mW to 300 W for the CW laser and 1 mJ/pulse to 1 J/pulse for the pulse laser. Preferably, the pulse laser is 32 mJ/pulse to 1 J/pulse and the CW laser has the power from 10 W to 300 W. When the CW is less than 10 mW and the pulse laser is less than 1 mJ/pulse, an energy sufficient to disrupt cells is not transferred. When the CW is greater than 300 W and the pulse laser is greater than 1 J/pulse, DNA is damaged.

In an embodiment of the present invention, a laser beam should be generated in a specific wavelength range which allows the magnetic beads to absorb the laser beam. The laser beam is generated preferably in the wavelength range of 750 nm or more, and more preferably 750-5000 nm. A laser absorption by the silica bead is increased at a wavelength less than 750 nm and a laser absorption by the solution is increased at a wavelength greater than 5000 nm. Thus, a distinct difference in laser absorption is not obtained. The laser beam can also be generated in one or more wavelength ranges. That is, the laser beam can have one wavelength or two or more different wavelengths within the above range.

In an embodiment of the present invention, the size of the magnetic bead is preferably from 50 nm to 1,000 µm, and more preferably, from 1 µm to 50 µm. When the size of the magnetic bead is less than 50 nm, physical and mechanical shocks are insufficient to cause cell lysis. When the size of the magnetic bead is greater than 1,000 µm, it is not suitable for LOC. The magnetic beads can also be a mixture of beads with two or more sizes. That is, the magnetic beads can have equal sizes to each other or be a mixture of beads with different sizes.

In an embodiment of the present invention, the capillary-shaped container can have a ratio of diameter to length ranging from 1:2 to 1:50 and have a diameter ranging from 1 nm to 5 mm. A phase containing beads is non-specifically bound to a glass wall, which occurs efficiently in a capillary with a limited diameter. Thus, if a container has a dimension outside the above range, a phase separation does not effectively occur, thereby resulting in a reduced purification effect.

In an embodiment of the present invention, the container can be composed of a material selected from the group consisting of polymers, organic materials, silicon, glass and metals. The container can be composed of any material capable of effectively fixing beads.

In an embodiment of the present invention, the magnetic bead can be any material which is magnetized. In particular, the magnetic beads preferably include at least one material selected from the group consisting of ferromagnetic Fe, Ni, Cr and oxides thereof.

In an embodiment of the present invention, the magnetic beads may be polymers, organic materials, silicon or glass coated with a ferromagnetic metal.

In an embodiment of the present invention, the surface of the magnetic bead is preferably negatively charged so that DNA cannot be attached thereto. The negative charge can be COO⁻, etc. Since DNA is negatively charged, it does not attach to the magnetic bead, which is negatively charged as well, due to a repulsive force. When DNA is attached to the magnetic bead, it is difficult to separate the DNA from the magnetic bead after cells are disrupted, which makes DNA purification more difficult.

In an embodiment of the present invention, the solution can be selected from the group consisting of saliva, urine, blood, serum and cell cultures. The solution can be any solution having nucleic acids, such as animal cells, plant cells, bacteria, viruses, phage and the like.

In an embodiment of the present invention, the solid support can include a silica bead, a silicon substrate, germanium, diamond, quartz, silicone, etc. The solid support should absorb no or a little laser beam at a near-infrared wavelength and can any support that allows nucleic acids to be bound thereto. A silica bead or silicon substrate is preferably used. The size of the silica bead may be from 50 nm to 1,000 µm, and preferably 1-50 µm. If the size of the silica bead is less than 50 nm, manufacturing costs increase. If the size is greater than 1,000 µm, it is suitable for LOC.

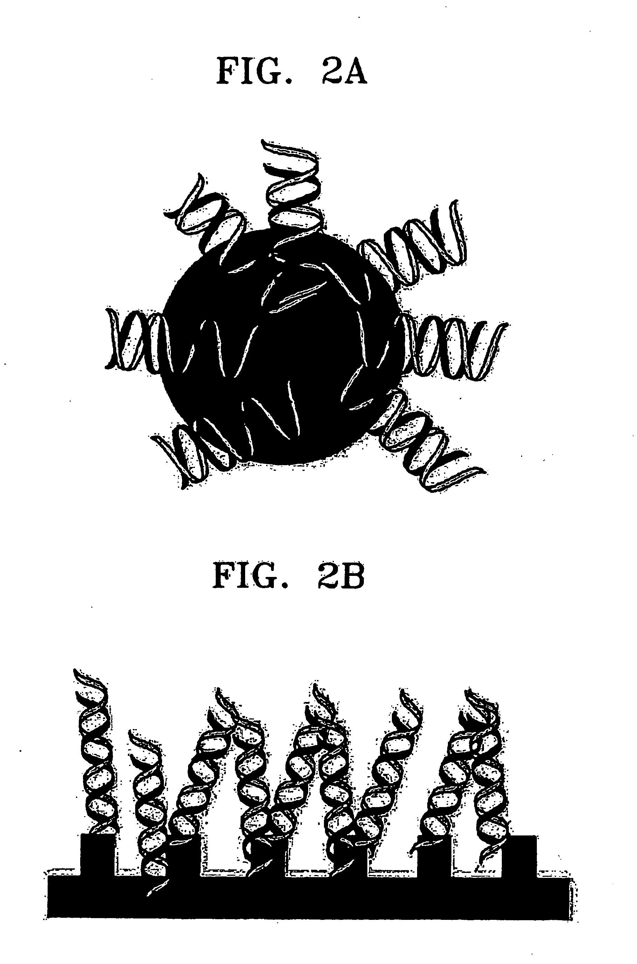

In an embodiment of the present invention, the silica bead may be a mixture of beads having two or more different sizes. That is, the silica beads can have equal sizes to each other or be a mixture of beads having different sizes. The surface of the silica bead may be coated with a positively-charged material in order to bind nucleic acids to the solid support by electrostatic interaction since nucleic acids are negatively-charged. A positively-charged material may be betaine, amino group, etc. FIG. 2A is a schematic diagram of a betaine-coated silica bead which captures nucleic acids.

In an embodiment of the present invention, the silicon substrate may be, for example, in a pillar form or have silica beads fixed thereto. These structures allow more nucleic acids to be bound to the silicon substrate due to increased surface area compared to a conventional silicon substrate. FIG. 2B is a schematic diagram of a betaine-coated silicon substrate in a pillar form, which captures nucleic acids. Small pieces of a silicon substrate can substitute silica beads or the capillary wall can be manufactured using a silicon substrate.

The present invention will now be described in greater detail with reference to the following examples. The following examples are for illustrative purposes only and are not intended to limit the scope of the invention.

### Examples

### Example 1: Purification of nucleic acids using the apparatus and method of

### the present invention

Nucleic acids from rHBV were purified using the apparatus and method for the purification of nucleic acids of the present invention. Specifically, as illustrated in FIG. 1, rHBV (30 µℓ), serum (5 µℓ), PBS (25 µℓ), betaine silanized silica beads (30 µℓ), and micro magnetic beads (30 µℓ, Dynabeads^{®} M-270 Carboxylic Acid, DYNAL, Norway) were mixed in a Lightcycler capillary (inner diameter: 2.42 mm, height: 35.40 mm, ratio of inner diameter:height=1:14.63). 808 nm, 21.1 W high power laser beam (HLU25F100-808, LIMO, Germany) was applied to disrupt viruses for 15 sec while stirring the capillary by vortexing. After lysing viruses, the virus lysate was wasted through a waste pump and silica beads capturing nucleic acids were washed with 120 µℓ of a 0.1 M PBS. Next, nucleic acids were eluted from the silica beads using 30 µℓ of a 0.1 N NaOH and neutralized with 1 µℓ of a 1M Tris buffer (pH 7), and then used in PCR amplification.

PCR was performed using primers as follows: primer TMP5-F (SEQ ID No: 1); and primer TMP5-R (SEQ ID No: 2). The primer pair was sites corresponding to 2,269-2,387 nucleotides of HBV genome. PCR amplification was carried out using Taq polymerase (Takara, Korea) for 40 cycles (pre-denaturation at 50°C for 10 minutes and at 95°C for 1 minute, denaturation at 95°C for 5 seconds, and annealing and extension at 62°C for 15 seconds). The amplified DNAs were analyzed in an Agilent BioAnalyzer 2100 (Agilent Technologies, Palo Alto, CA) using a commercially available DNA 500 assay sizing reagent sets.

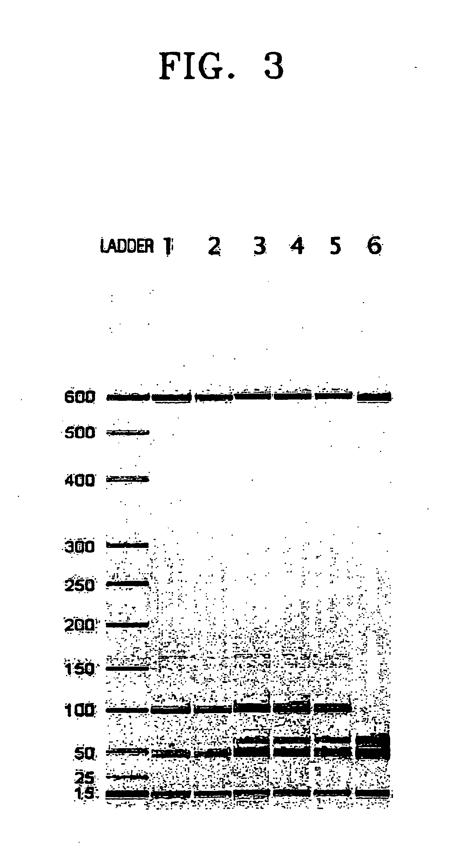

FIG. 3 illustrates the results of electrophoresis of PCR products according to DNA purification methods. The upper arrow designates a band of the desired PCR product and the lower arrow designates a dimmer of PCR primer as a PCR side product. Lanes 1 and 2 are the results of performing PCR after purifying nucleic acids according to the method of the present invention and lanes 3 to 5 are the results of performing PCR after purifying nucleic acids using Qiagen Ultrasense kit, as PCR positive controls. Sample 6 is a negative control and the results of performing PCR using only distilled water. Compositions of the respective samples are given in Table below.

**Table**

| Sample | Serum (µℓ) | PBS (µℓ) | Betaine silanized silica beads (µℓ) | Micro magnetic bead (µℓ) | rHBV (µℓ) |
|---|---|---|---|---|---|
| 1 | 5 | 25 | 30 | 30 | 30 |
| 2 | 5 | 25 | 30 | 30 | 30 |
| 3 | 200 | 700 | - | - | 100 |
| 4 | 200 | 700 | - | - | 100 |
| 5 | 200 | 700 | - | - | 100 |
| 6 | Distilled water | | | | |

As can be seen from FIG. 3, in the negative control (lane 6), a PCR product was not observed as had been expected, but in the case of the present invention (lanes 1 and 2), PCR products were observed at the desired position (100 bp). In the case of performing PCR after purifying nucleic acids using Qiagen Ultrasense kit (lanes 3 to 5), PCR products were also observed. Consequently, it can be seen that the positive control requiring long time and many steps for purification, where DNA was purified using Qiagen Ultrasense kit, and the method of the present invention showed similar PCR efficiency. Since the method of the present invention effectively performs PCR in shorter time and less steps than the conventional Qiagen method for DNA purification without PCR inhibition, it can be usefully applied to LOC.

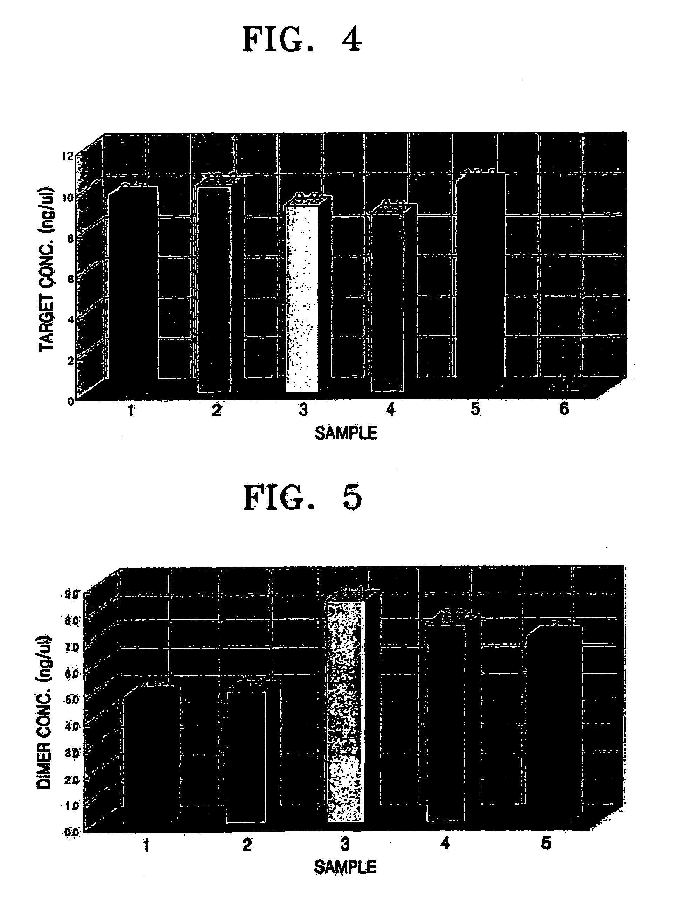

FIG. 4 illustrates the concentrations of amplified PCR products according to DNA purification methods. Bars indicate amplified DNA concentration (ng/µℓ). The amounts of PCR products were quantified with Agilent BioAnalyzer 2100. Samples 1 and 2 were PCR products after purifying nucleic acids according to the method of the present invention, Samples 3 to 5 were PCR positive controls where PCR was carried out after purifying nucleic acids using Qiagen Ultrasense kit, and Sample 6 was a PCR negative control where PCR was performed using only distilled water. Compositions of the respective samples are the same as in the above Table. As shown in FIG. 4, the results of PCR using the method of the present invention is similar to or superior over the results of using Qiagen Ultrasense kit.

FIG. 5 illustrates the concentrations of dimers produced by PCR. The bars indicate dimer concentration (ng/µℓ). The amounts of dimers were quantified with Agilent BioAnalyzer 2100. The respective sample Nos. are the same as in FIG. 4. The dimer is a side product of PCR. Generally, when the results of PCR were good, the concentration of the desired PCR product increases and the concentration of the dimer decreases. When the results of PCR were poor, the concentration of the desired PCR product decreases and the concentration of the dimer increases.
Thus, as a primer DNA is purified, the concentration of the desired PCR product increases and the concentration of the dimer decreases. As shown in FIG. 5, the method of the present invention (Samples 1 and 2) has a lower amount of the dimmer than the Qiagen method (Samples 3 to 5), indicating that the method of the present invention provides more effectively purified template DNA for PCR amplification than the Qiagen method. Thus, better PCR yield is expected upon optimizing the method of the present invention.

As described above, according to the method of the present invention, PCR inhibitors can be removed to increase PCR yield and nucleic acids can be purified using a silicon substrate or silica beads. Thus, the method of the present invention can be applied to LOC fabrication.
## Claims

1. A nucleic acid purification apparatus for cells or viruses, comprising:
a cell lysis capillary having a sample inlet through which samples, magnetic beads, and a solid support, to which nucleic acids conbebound, are introduced;
a vibrator attached to the capillary and mixing the samples, magnetic beads, and solid support in the capillary;
a laser generator attached to the capillary and irradiating a laser beam onto the capillary;
a magnetic force generator attached to the capillary and fixing the magnetic beads to a capillary wall;
a waste chamber attached to the capillary and discharging a lysate;
an elution buffer chamber attached to the capillary and eluting nucleic acids from the solid support having nucleic acids bound thereto; and
a neutralization buffer chamber attached to the capillary and supplying a neutralization buffer for neutralizing an eluted nucleic acid solution.

2. The apparatus of claim 1, wherein the vibrator is selected from the group consisting of sonicators, vibrators using a magnetic field, vibrators using an electric field, and mechanical vibrators.

3. The apparatus of claim 1, wherein the magnetic force generator is located above a laser pathway and is an electromagnet which is turned on when the magnetic beads in the cell lysis capillary are boiled.

4. The apparatus of claim 1, further comprising a DNA amplification chamber connected to the cell lysis capillary through a channel which is opened or closed by a valve.

5. The apparatus of claim 1, further comprising a membrane which is located in a channel disposed between the cell lysis capillary and the DNA amplification chamber and filters the solid support.

6. The apparatus of claim 1, further comprising a washing buffer chamber attached to the capillary and washing the solid support having nucleic acids bound thereto.

7. A method of purifying nucleic acids using the nucleic acid purification apparatus of claim 1, the method comprising:
injecting a solution containing cells or viruses in a capillary- shaped container containing magnetic beads and a solid support;
operating a vibrator to mix the solution, the magnetic beads and the solid support;
irradiating a laser beam onto the magnetic beads to disrupt the cells or viruses and binding compounds in the resulting cell or virus lysate to the magnetic beads and binding nucleic acids in the lysate to the solid support;
fixing the magnetic beads, to which the compounds in the cell or virus lysate are bound, to a capillary-shaped container wall by means of a magnetic force generator;
discharging the lysate which contains no magnetic bead; and
eluting nucleic acids from the solid support and neutralizing them.

8. A method of continuously performing purification and amplification of nucleic acids using the nucleic acid purification apparatus of claim 4, the method comprising:
injecting a solution containing cells or viruses to a capillary-shaped container containing magnetic beads and a solid support;
operating a vibrator to mix the solution, the magnetic beads, and the solid support;
irradiating a laser beam onto the magnetic beads to disrupt the cells or viruses and binding compounds in the resulting cell or virus lysate to the magnetic beads and binding nucleic acids in the lysate to the solid support;
fixing the magnetic beads, to which the compounds in the cell or virus lysate are bound, to a capillary-shaped container wall by means of a magnetic force generator;
discharging the lysate which contains no magnetic bead;
eluting the nucleic acids from the solid support and neutralizing an eluted nucleic acid solution; and
obtaining a solution that contains nucleic acids and transferring the resulting solution to a amplification chamber through a channel connecting the container and the amplification chamber to perform amplification.

9. The method of claim 7 or 8, further comprising washing the solid support to which nucleic acids are bound and discharging a washing solution after discharging the lysate.

10. The method of claim 7 or 8, wherein the laser comprises a pulse laser or continuous wave (CW) laser.

11. The method of claim 10, wherein the pulse laser is 1 mJ/pulse to 1 J/pulse and the CW laser has a power of 10 mW to 300 W.

12. The method of claim 7 or 8, wherein the laser beam is generated in a wavelength range of from 750 nm to 5000 nm.

13. The method of claim 12, wherein the laser beam is generated in one or more wavelength ranges.

14. The method of claim 7 or 8, wherein the size of the magnetic bead is from 50 nm to 1,000 µm.

15. The method of claim 14, wherein the magnetic beads are a mixture of beads having two or more sizes.

16. The method of claim 7 or 8, wherein the capillary-shaped container has a ratio of diameter to length ranging from 1:2 to 1:50.

17. The method of claim 16, wherein the container has a diameter ranging from 1 nm to 5 mm.

18. The method of claim 7 or 8, wherein the container is composed of a material selected from the group consisting of polymers, organic materials, silicon, glass and metals.

19. The method of claim 7 or 8, wherein the magnetic beads comprise at least one material selected from the group consisting of ferromagnetic Fe, Ni, Cr, and oxides thereof.

20. The method of claim 7 or 8, wherein the magnetic beads are polymers, organic materials, silicon or glass coated with a ferromagnetic metal.

21. The method of claim 7 or 8, wherein the magnetic beads have a negatively charged surface.

22. The method of claim 7 or 8, wherein the solution is selected from the group consisting of saliva, urine, blood, serum and cell cultures.

23. The method of claim 7 or 8, wherein the solid support is selected from the group consisting of silica beads, a silicon substrate, germanium, diamond, quartz, and silicone.

24. The method of claim 23, wherein the size of the silica bead is 50 nm to 1,000 µm.

25. The method of claim 24, wherein the silica beads are a mixture of beads having two or more sizes.

26. The method of claim 24, wherein the silica bead is coated with a positively-charged material.

27. The method of claim 23, wherein the silicon substrate is in a pillar form or has silica beads fixed thereto.

## Patentansprüche

1. Gerät zum Aufreinigen von Nukleinsäure aus Zellen oder Viren, umfassend:
eine Zelllysekapillare mit einem Probeneinlass, durch den Proben, magnetische Kügelchen und ein fester Träger, an den Nukleinsäure gebunden werden kann,
eingeführt werden;
einen Rüttler, welcher der Kapillare zugeordnet ist und der die Proben, magnetischen Kügelchen und den festen Träger in der Kapillare mischt;
einen Lasergenerator, welcher der Kapillare zugeordnet ist und der einen Laserstrahl auf die Kapillare ausstrahlt;
einen Magnetfeldgenerator, welcher der Kapillare zugeordnet ist und der die magnetischen Kügelchen an der Kapillarwand fixiert;
eine Abfallkammer, die der Kapillare zugeordnet ist und in die das Lysat abgelassen wird;
eine Elutionspufferkammer, die der Kapillare zugeordnet ist und welche die Nukleinsäuren von dem festen Träger, an den Nukleinsäuren gebunden sind, eluiert;
und
eine Neutralisationspufferkammer, die der Kapillare zugeordnet ist und die einen Neutralisationspuffer zum Neutralisieren einer eluierten Nukleinsäurelösung liefert.

2. Gerät nach Anspruch 1, wobei der Rüttler ausgewählt ist aus der Gruppe bestehend aus einer Beschallungsvorrichtung, Rüttlern, die ein Magnetfeld verwenden, Rüttlern, die ein elektrisches Feld verwenden, und mechanischen Rüttlern.

3. Gerät nach Anspruch 1, wobei der Magnetfeldgenerator oberhalb eines Laserpfades angeordnet ist und ein Elektromagnet ist, der angeschaltet wird, wenn die magnetischen Kügelchen in der Zelllysekapillare gesiedet werden.

4. Gerät nach Anspruch 1, ferner umfassend eine DNA-Amplifikationskammer, die mit der Zelllysekapillare durch einen Kanal, der mittels eines Ventils geöffnet oder geschlossen werden kann, verbunden ist.

5. Gerät nach Anspruch 1, ferner umfassend eine Membran, die in einem Kanal angeordnet ist, der sich zwischen der Zelllysekapillare und der DNA-Amplifikationskammer erstreckt, und den festen Träger herausfiltert.

6. Gerät nach Anspruch 1, ferner umfassend eine Waschpufferkammer, die der Kapillare zugeordnet ist und die den festen Träger, an den die Nukleinsäure gebunden ist, wäscht.

7. Verfahren zum Aufreinigen von Nukleinsäure unter Verwendung des Gerätes zur Aufreinigung von Nukleinsäure nach Anspruch 1, wobei das Verfahren umfasst:
Einspeisen einer Lösung enthaltend Zellen oder Viren in einen kapillarförmigen Behälter, der magnetische Kügelchen und einen festen Träger enthält;
Betätigen eines Rüttlers zum Mischen der Lösung, der magnetischen Kügelchen und des festen Trägers;
Ausstrahlen eines Laserstrahls auf die magnetischen Kügelchen, um die Zellen oder die Viren aufzuschließen und Bestandteile aus dem entstehenden Zell- oder Virenlysat an die magnetischen Kügelchen zu binden und die Nukleinsäuren aus dem Lysat an den festen Träger zu binden;
Fixieren der magnetischen Kügelchen, an welche die Bestandteile aus dem Zell- oder Viruslysat gebunden sind, an die Wand des kapillarförmigen Behälters mittels eines Magnetfeldgenerators;
Ablassen des Lysates, das keine magnetischen Kügelchen enthält; und
Eluieren der Nukleinsäuren von dem festen Träger und Neutralisieren derselben.

8. Verfahren zum Durchführen der Aufreinigung und Amplifikation von Nukleinsäuren, ohne Unterbrechung, unter Verwendung des Gerätes zur Nukleinsäureaufreinigung von Anspruch 4, wobei das Verfahren umfasst:
Einspeisen einer Lösung enthaltend Zellen oder Viren in einen kapillarförmigen Behälter, der magnetische Kügelchen und einen festen Träger enthält;
Betätigen eines Rüttlers zum Mischen der Lösung, der magnetischen Kügelchen und des festen Trägers;
Ausstrahlen eines Laserstrahls auf die magnetischen Kügelchen, um die Zellen oder Viren aufzuschließen und Bestandteile in dem entstehenden Zell- oder Virenlysat an die magnetischen Kügelchen zu binden und die Nukleinsäuren aus dem Lysat an den festen Träger zu binden;
Fixieren der magnetischen Kügelchen, an welche die Bestandteile aus dem Zell- oder Viruslysat gebunden sind, an die Wand des kapillarförmigen Behälters mittels eines Magnetfeldgenerators;
Ablassen des Lysates, das keine magnetischen Kügelchen enthält;
Eluieren der Nukleinsäuren von dem festen Träger und Neutralisieren einer eluierten Nukleinsäurelösung; und
Erhalten einer Lösung, die Nukleinsäuren enthält und Übertragen der entstandenen Lösung in eine Amplifikationskammer durch einen Kanal, welcher den Behälter und die Amplifikationskammer verbindet, um die Amplifikation durchzuführen.

9. Verfahren nach Anspruch 7 oder 8, ferner umfassend das Waschen des festen Trägers, an den Nukleinsäuren gebunden sind, und Ablassen einer Waschlösung, nachdem das Lysat abgelassen wurde.

10. Verfahren nach Anspruch 7 oder 8, wobei der Laser einen gepulsten Laser oder einen kontinuierlichen (CW) Laser umfasst.

11. Verfahren nach Anspruch 10, wobei der gepulste Laser 1 mJ/Puls bis 1 J/Puls ausstrahlt und der kontinuierliche Laser eine Leistung von 10 mW bis 300 W hat.

12. Verfahren nach Anspruch 7 oder 8, wobei der Laserstrahl erzeugt wird im Wellenlängenbereich von 750 nm bis 5000 nm.

13. Verfahren nach Anspruch 12, wobei der Laserstrahl in einem oder mehreren Wellenlängenbereichen erzeugt wird.

14. Verfahren nach Anspruch 7 oder 8, wobei die Größe der magnetischen Kügelchen von 50 nm bis 1000 µm liegt.

15. Verfahren nach Anspruch 14, wobei die magnetischen Kügelchen ein Gemisch aus Kügelchen mit zwei oder mehr Größen sind.

16. Verfahren nach Anspruch 7 oder 8, wobei der kapillarförmige Behälter ein Verhältnis von Durchmesser zu Länge im Bereich von 1:2 bis 1:50 hat.

17. Verfahren nach Anspruch 16, wobei der Behälter einen Durchmesser im Bereich von 1 nm bis 5 nm hat.

18. Verfahren nach Anspruch 7 oder 8, wobei der Behälter aus einem Material besteht, das ausgewählt ist aus der Gruppe bestehend aus Polymeren, organischen Materialien, Silikon, Glass und Metallen.

19. Verfahren nach Anspruch 7 oder 8, wobei die magnetischen Kügelchen wenigstens ein Material umfassen, das ausgewählt ist aus der Gruppe bestehend aus ferromagnetischem Fe, Ni, Cr, und Oxiden dieser Elemente.

20. Verfahren nach Anspruch 7 oder 8, wobei die magnetischen Kügelchen Polymere, organische Materialien, Silikon oder Glas sind, die mit einem ferromagnetischen Metall beschichtet sind.

21. Verfahren nach Anspruch 7 oder 8, wobei die magnetischen Kügelchen eine negativ geladene Oberfläche aufweisen.

22. Verfahren nach Anspruch 7 oder 8, wobei die Lösung ausgewählt ist aus der Gruppe bestehend aus Speichel, Urin, Blut, Serum und Zellkulturen.

23. Verfahren nach Anspruch 7 oder 8, wobei der feste Träger ausgewählt ist aus der Gruppe bestehend aus Silika-Kügelchen, einem Silikonsubstrat, Germanium, Diamant, Quarz und Silikon.

24. Verfahren nach Anspruch 23, wobei die Größe der Silika-Kügelchen 50 nm bis 1000 µm ist.

25. Verfahren nach Anspruch 24, wobei die Silika-Kügelchen ein Gemisch aus Kügelchen mit zwei oder mehr Größen sind.

26. Verfahren nach Anspruch 24, wobei die Silika-Kügelchen mit einem positiv geladenen Material beschichtet sind.

27. Verfahren nach Anspruch 23, wobei das Silikonsubstrat eine Säulenform aufweist oder Silika-Kügelchen daran fixiert sind.

## Revendications

1. Appareil de purification d'acide nucléique pour cellules et virus, comprenant :
un capillaire de lyse cellulaire possédant un orifice d'entrée d'échantillon par lequel des échantillons, des billes magnétiques, et un support solide, auquel des acides nucléiques peuvent être liés, sont introduits ;
un dispositif vibrant attaché au capillaire et mélangeant les échantillons, les billes magnétiques, et le support solide dans le capillaire ;
un générateur laser attaché au capillaire et émettant un faisceau laser irradiant le capillaire ;
un générateur de force magnétique attaché au capillaire et immobilisant les billes magnétiques sur une paroi du capillaire ;
une chambre à déchets attachée au capillaire et évacuant un lysat ;
une chambre à tampon d'élution attachée au capillaire et éluant des acides nucléiques à partir du support solide auquel des acides nucléiques sont liés ; et
une chambre à tampon de neutralisation attachée au capillaire et fournissant un tampon de neutralisation permettant la neutralisation d'une solution d'acides nucléiques élués.

2. Appareil selon la revendication 1, dans lequel le dispositif vibrant est choisi dans le groupe constitué des sonicateurs, des vibreurs utilisant un champ magnétique, des vibreurs utilisant un champ électrique, et des vibreurs mécaniques.

3. Appareil selon la revendication 1, dans lequel le générateur de force magnétique est situé au-dessus d'un chemin du laser et comprend un électro-aimant qui est allumé lorsque les billes magnétiques présentes dans le capillaire de lyse cellulaire sont mises en ébullition.

4. Appareil selon la revendication 1, comprenant en outre une chambre d'amplification d'ADN reliée au capillaire de lyse cellulaire par l'intermédiaire d'un canal qui est ouvert ou fermé par une soupape.

5. Appareil selon la revendication 1, comprenant en outre une membrane qui est située dans un canal disposé entre le capillaire de lyse cellulaire et la chambre d'amplification d'ADN et qui filtre le support solide.

6. Appareil selon la revendication 1, comprenant en outre une chambre à tampon de lavage attachée au capillaire et lavant le support solide auquel des acides nucléiques sont liés.

7. Procédé de purification d'acides nucléiques à l'aide de l'appareil de purification d'acide nucléique selon la revendication 1, ledit procédé comprenant :
injecter une solution contenant des cellules ou des virus dans un récipient en forme de capillaire contenant des billes magnétiques et un support solide ;
actionner un dispositif vibrant pour mélanger la solution, les billes magnétiques et le support solide ;
irradier les billes magnétiques avec un faisceau laser pour rompre les cellules ou les virus, et provoquer la liaison de composés présents dans le lysat cellulaire ou viral résultant aux billes magnétiques et la liaison d'acides nucléiques présents dans le lysat au support solide ;
immobiliser les billes magnétiques, auxquelles les composés présents dans le lysat cellulaire ou viral sont liés, sur un récipient en forme de capillaire au moyen d'un générateur de force magnétique ;
évacuer le lysat qui ne contient aucune bille magnétique ; et
éluer les acides nucléiques du support solide et neutraliser ceux-ci.

8. Procédé de purification et d'amplification d'acides nucléiques en continu à l'aide de l'appareil de purification d'acide nucléique selon la revendication 4, ledit procédé comprenant :
injecter une solution contenant des cellules ou des virus dans un récipient en forme de capillaire contenant des billes magnétiques et un support solide ;
actionner un dispositif vibrant pour mélanger la solution, les billes magnétiques, et le support solide ;
irradier les billes magnétiques avec un faisceau laser pour rompre les cellules ou les virus, et provoquer la liaison de composés présents dans le lysat cellulaire ou viral résultant aux billes magnétiques et la liaison d'acides nucléiques présents dans le lysat au support solide ;
immobiliser les billes magnétiques, auxquelles les composés présents dans le lysat cellulaire ou viral sont liés, sur un récipient en forme de capillaire au moyen d'un générateur de force magnétique ;
évacuer le lysat qui ne contient aucune bille magnétique ;
éluer les acides nucléiques du support solide et neutraliser une solution d'acides nucléiques élués ; et
obtenir une solution qui contient des acides nucléiques et transférer la solution résultante dans une chambre d'amplification à travers un canal reliant le récipient et la chambre d'amplification afin d'accomplir une amplification.

9. Procédé selon la revendication 7 ou 8, consistant en outre à laver le support solide auquel des acides nucléiques sont liés et à évacuer une solution de lavage après évacuation du lysat.

10. Procédé selon la revendication 7 ou 8, dans lequel le laser comprend un laser à impulsions ou un laser à onde continue (CW).

11. Procédé selon la revendication 10, dans lequel le laser à impulsions a une énergie de 1 mJ/impulsion à 1 J/impulsion et le laser CW a une puissance de 10 mW à 300 W.

12. Procédé selon la revendication 7 ou 8, dans lequel le faisceau laser est généré dans une gamme de longueurs d'onde de 750 nm à 5000 nm.

13. Procédé selon la revendication 12, dans lequel le faisceau laser est généré dans une ou plusieurs gammes de longueurs d'onde.

14. Procédé selon la revendication 7 ou 8, dans lequel la taille de la bille magnétique est de 50 nm à 1000 µm.

15. Procédé selon la revendication 14, dans lequel les billes magnétiques sont un mélange de billes ayant deux ou plusieurs tailles.

16. Procédé selon la revendication 7 ou 8, dans lequel le récipient en forme de capillaire a un rapport diamètre/longueur allant de 1:2 à 1:50.

17. Procédé selon la revendication 16, dans lequel le récipient a un diamètre allant de 1 nm à 5 mm.

18. Procédé selon la revendication 7 ou 8, dans lequel le récipient se compose d'un matériau choisi dans le groupe constitué des polymères, des matériaux organiques, du silicium, du verre et des métaux.

19. Procédé selon la revendication 7 ou 8, dans lequel les billes magnétiques comprennent au moins un matériau choisi dans le groupe constitué des Fe, Ni, Cr ferromagnétiques, et des oxydes de ceux-ci.

20. Procédé selon la revendication 7 ou 8, dans lequel les billes magnétiques sont en polymères, matériaux organiques, silicium ou verre, revêtu(s) d'un métal ferromagnétique.

21. Procédé selon la revendication 7 ou 8, dans lequel les billes magnétiques ont une surface chargée négativement.

22. Procédé selon la revendication 7 ou 8, dans lequel la solution est choisie dans le groupe constitué de la salive, de l'urine, du sang, du sérum et des cultures cellulaires.

23. Procédé selon la revendication 7 ou 8, dans lequel le support solide est choisi dans le groupe constitué des billes de silice, d'un substrat en silicium, du germanium, du diamant, du quartz, et d'un silicone.

24. Procédé selon la revendication 23, dans lequel la taille de la bille de silice est de 50 nm à 1000 µm.

25. Procédé selon la revendication 24, dans lequel les billes de silice sont un mélange de billes ayant deux ou plusieurs tailles.

26. Procédé selon la revendication 24, dans lequel la bille de silice est revêtue d'un matériau chargé positivement.

27. Procédé selon la revendication 23, dans lequel le substrat en silicium se présente sous forme de pilier ou possède des billes de silice fixées sur lui.
